# EUROPÄISCHE PATENTSCHRIFT

(11) **EP 2 867 336 B1**
(45) Veröffentlichungstag und Bekanntmachung des Hinweises auf die Patenterteilung: **04.11.2015**
(21) Anmeldenummer: 13744973.2
(22) Anmeldetag: 01.08.2013
(51) Int. Cl.: C10G 9/36

(54) **VERFAHREN ZUR UMSETZUNG VON KOHLENWASSERSTOFFEINSÄTZEN DURCH THERMISCHES DAMPFSPALTEN**
METHOD FOR CONVERTING HYDROCARBON FEEDSTOCKS BY MEANS OF THERMAL STEAM CRACKING
PROCÉDÉ DE CONVERSION DE CHARGES HYDROCARBONÉES PAR CRAQUAGE THERMIQUE À LA VAPEUR D'EAU

(30) Priorität: 09.08.2012 EP 12005780
(43) Veröffentlichungstag der Anmeldung: 06.05.2015
(73) Patentinhaber: Linde AG, 80331 München (DE)
(72) Erfinder: SCHMIDT, Gunther, 82041 Deisenhofen (DE); FRITZ, Helmut, 81375 München (DE); WALTER, Stefanie, 82418 Seehausen (DE)
(86) Internationale Anmeldenummer: PCT/EP2013/002295
(87) Internationale Veröffentlichungsnummer: WO 2014/023406

(56) Entgegenhaltungen:
- FR-A- 1 196 927
- GB-A- 1 357 495
- US-A1- 2004 209 964
- US-A1- 2008 194 900
- US-A1- 2008 223 754

## Beschreibung

Die vorliegende Erfindung betrifft ein Verfahren zur Umsetzung von Kohlenwasserstoffeinsätzen durch thermisches Dampfspalten zu mindestens einem olefinhaltigen Produktstrom, welcher zumindest Ethylen und Propylen enthält, wobei ein erster Kohlenwasserstoffeinsatz in mindestens einem ersten Spaltofen und ein zweiter Kohlenwasserstoffeinsatz in mindesten einem zweiten Spaltofen wenigstens teilweise umgesetzt wird.

Beim thermischen Dampfspalten (auch als Dampfcracken oder Steamcracken bezeichnet, engl. Steam Cracking) handelt es sich um ein seit langem etabliertes Verfahren der Petrochemie. Die klassische Zielverbindung beim thermischen Dampfspalten ist das Ethylen (auch: Ethen), das eine wichtige Ausgangsverbindung für eine Reihe chemischer Synthesen darstellt.

Als Einsatz für das thermische Dampfspalten können sowohl Gase wie Ethan, Propan oder Butan und entsprechende Gemische als auch flüssige Kohlenwasserstoffe, wie beispielsweise Naphtha, und Kohlenwasserstoffgemische verwendet werden.

Zu den beim thermischen Dampfspalten im Einzelnen verwendeten Vorrichtungen und Reaktionsbedingungen und zu den ablaufenden Reaktionen sowie zu Einzelheiten der Raffinerietechnik sei auf entsprechende Artikel in Nachschlagewerken wie Zimmermann, H. und Walzl, R.: Ethylene. In: Ullmann's Encyclopedia of Industrial Chemistry. 6. Aufl. Weinheim: Wiley-VCH, 2005, und Irion, W.W. und Neuwirth, O.S.: Oil Refining. In: Ullmann's Encyclopedia of Industrial Chemistry. 6. Aufl. Weinheim: Wiley-VCH 2005, verwiesen. Verfahren zur Herstellung von Olefinen sind beispielsweise auch in der US 3 714 282 A und der US 6 743 961 B1 offenbart. Aus der US 2008/0223754 ist beispielweise bekannt, dass in Raffinerien Cracker, wie beipsielsweise Hydrocracker, katalytische Cracker, FCC-Cracker oder thermische Dampfcracker zur Verarbeitungen von Kohlenwasserstoffschnitten eingesetzt werden können.

Zum thermischen Dampfspalten werden Spaltöfen eingesetzt. Die Spaltöfen sind, zusammen mit Quechenheit und nachgeschalteten Einrichtungen zur Aufarbeitung der gebildeten Produktgemische, in entsprechenden größeren Anlagen zur Olefinherstellung integriert, die im Rahmen dieser Anmeldung als "Steamcracker" bezeichnet werden.

Eine wichtige Kenngröße beim thermischen Dampfspalten ist die sogenannte Spaltschärfe (engl. Cracking Severity), welche die Spaltbedingungen bestimmt. Die Spaltbedingungen werden insbesondere beeinflusst von der Temperatur und der Verweilzeit sowie der Partialdrücke der Kohlenwasserstoffe und des Wasserdampfs. Auch die Zusammensetzung der als Einsatz verwendeten Kohlenwasserstoffgemische und die Bauart der verwendeten Spaltöfen beeinflussen die Spaltbedingungen. Aufgrund der wechselseitigen Einflüsse dieser Faktoren wird die Spaltbedingung normalerweise über das Verhältnis von Propylen (auch als Propen bezeichnet) zu Ethylen im Spaltgas festgelegt.

Beim thermischen Dampfspalten entstehen je nach Einsatzgemisch und Spaltbedingungen neben der klassischen Zielverbindung Ethylen mitunter beträchtliche Mengen an Nebenprodukten, die aus einem entsprechenden Produktstrom abgetrennt werden können. Hierbei handelt es sich unter anderem um niedere Alkene wie z.B. Propylen und Butene sowie Diene, wie beispielsweise Butadiene, und sowie Aromaten wie z.B. Benzol, Toluol und Xylole. Diese besitzen einen vergleichsweise hohen wirtschaftlichen Wert, so dass ihre Bildung als sogenannte Mehrwertprodukte (engl. High Value Products) erwünscht ist.

Die US2008/0194900 A1 offenbart ein Verfahren zur Erzeugung von Olefinen durch Dampfspaltung von aromatischen Naphtha, bei dem gebildetes Ethan, Propan und ein gebildeter C₅-Olefinstrom in die Spaltung zurückgeführt werden können.

Die US 6 743 961 B2 offenbart ein Verfahren zur Erzeugung von Olefinen, bei dem Rohöl in einer kombinierten Verdampfungs- und Spalteinheit teilweise verdampft wird. Der gebildete Dampf und die verbleibende Flüssigkeit werden bei unterschiedlichen Spaltbedingungen gespalten.

In der US 2004/209964 A1 wird ein Verfahren vorgeschlagen, bei dem ein Fischer-Tropsch-Produktstrom fraktioniert wird. Kohlenwasserstoffe unterschiedlicher Kettenlängen werden bei unterschiedlichen Spaltbedingungen gespalten.

Die vorliegende Erfindung stellt sich die Aufgabe, die Möglichkeiten zur Gewinnung von olefinhaltigen Produktgemischen aus Kohlenwasserstoffen durch thermisches Dampfspalten zu verbessern.

### Offenbarung der Erfindung

Die Erfindung schlägt vor diesem Hintergrund ein Verfahren zur Umsetzung von Kohlenwasserstoffeinsätzen durch thermisches Dampfspalten zu einem olefinhaltigen Produktstrom, welcher zumindest Ethylen und Propylen enthält, wobei ein erster Kohlenwasserstoffeinsatz in mindestens einem ersten Spaltofen und ein zweiter Kohlenwasserstoffeinsatz in mindesten einem zweiten Spaltofen wenigstens teilweise umgesetzt wird, mit den Merkmalen der unabhängigen Patentansprüche vor. Bevorzugte Ausgestaltungen sind Gegenstand der Unteransprüche und der nachfolgenden Beschreibung.

### Vorteile der Erfindung

Erfindungsgemäß wird ein Verfahren vorgeschlagen, bei welchem der zweite Kohlenwasserstoffeinsatz überwiegend Kohlenwasserstoffe mit einer Kohlenstoffzahl von 5 oder/und 4 enthält und größtenteils aus einer oder mehreren rückgeführten Fraktionen, welche aus dem Produktstrom gewonnen werden, besteht, wobei der zweite Kohlenwasserstoff im zweiten Spaltofen (2) mit Spaltbedingungen umgesetzt wird, die zu einem Verhältnis von Propylen zu Ethylen von 0,85 bis 1,6 kg/kg führen.

Im Rahmen der Erfindung werden als erster beziehungsweise zweiter Kohlenwasserstoffeinsatz alle Kohlenwasserstoffe bezeichnet, die in den ersten beziehungsweise in den zweiten Spaltofen geführt werden. Es wird also ein erster Kohlenwasserstoffeinsatz in einem ersten Spaltofen zumindest teilweise umgesetzt und ein zweiter Kohlenwasserstoffeinsatz in einem zweiten Spaltofen. Erfindungsgemäß besteht dabei der zweite Kohlenwasserstoffeinsatz vorteilhafterweise aus einer Fraktion oder aus mehreren Faktionen, die aus dem Produktstrom abgetrennt werden und in den zweiten Spaltofen, in welchem bei der zweiten Spaltschärfe der zweite Kohlenwasserstoffeinsatz umgesetzt wird, rückgeführt werden. Dem zweiten Kohlenwasserstoffeinsatz wird somit mit Vorteil kein Frischeinsatz zugegeben und dem zweiten Spaltofen wird vorteilhafterweise auch kein Frischeinsatz zugeführt.

Unter Spaltofen wird im Rahmen dieser Erfindung eine Spalteinheit verstanden, in der die Spaltbedingungen festgelegt sind. Es ist möglich, dass an einem Gesamtofen eine Unterteilung in zwei oder mehr Spaltöfen vorliegt. Man spricht dann häufig von Ofenzellen. Mehrere, zu einem Gesamtofen gehörende Ofenzellen weisen in der Regel voneinander unabhängige Strahlungszonen und eine gemeinsame Konvektionszone sowie einen gemeinsamen Rauchabzug auf. In diesen Fällen kann jede Ofenzelle mit eigenen Spaltbedingungen betrieben werden. Jede Ofenzelle ist somit eine Spalteinheit und wird infolgedessen hier als Spaltofen bezeichnet. Der Gesamtofen weist dann mehrere Spalteinheiten oder, anders ausgedrückt, er weist mehrere Spaltöfen auf. Liegt nur eine Ofenzelle vor, ist diese die Spalteinheit und somit der Spaltofen. Spaltöfen können zu Gruppen zusammengefasst werden, welche beispielsweise mit dem gleichen Einsatz versorgt werden. Die Spaltbedingungen innerhalb einer Ofengruppe werden in der Regel gleich oder ähnlich eingestellt werden.

Da der zweite Kohlenwasserstoffeinsatz erfindungsgemäß größtenteils aus rückgeführten Fraktionen besteht, ist die Zusammensetzung des zweiten Kohlenwasserstoffeinsatzes gut festgelegt. Dies gilt insbesondere im Vergleich zu Kohlenwasserstoffeinsätzen, die einen Frischeinsatz enthalten. Der zweite Kohlenwasserstoff wird nun in dem mindestens einen zweiten Spaltofen umgesetzt. Dies hat den Vorteil, dass Spaltbedingungen und Einsatz optimal aufeinander abgestimmt werden können. Wenn nun der zweite Kohlenwasserstoffeinsatz aus überwiegend Kohlenwasserstoffen mit einer Kohlenstoffzahl von 5 oder/und 4 besteht, kann dieser bei milden und sehr milden Bedingungen gespalten werden.

Hingegen entstehen bei der thermischen Spaltung von Kohlenwasserstoffen üblicher Zusammensetzung, wie beispielsweise Naphtha, unter milden Spaltbedingungen sehr große Mengen an Pyrolysebenzin, welches aufgrund der großen Menge sehr schwierig in der Handhabung wird. Dies ist ein Resultat der vergleichsweise geringeren Umsetzung des Einsatzes im Spaltofen bei milden Spaltbedingungen. Mit dem erfindungsgemäßen Verfahren wird erreicht, dass diese Probleme nicht auftreten.

Mit dem erfindungsgemäßen Verfahren wird es somit möglich, eine Anlage zum Steamcracken derartig zu betreiben, dass im Verhältnis zum Frischeinsatz mehr Propylen entsteht als in einer herkömmlichen Anlage, in welcher das erfindungsgemäße Verfahren nicht eingesetzt wird. Es erhöht sich also die Ausbeute an Propylen. Dies wird mit der Erfindung insbesondere dadurch erreicht, dass durch das gezielte Rückführen von Fraktionen eine Spaltung bei milden Spaltbedingungen vorteilhaft ausführbar wird.

Der Begriff "überwiegend" wird im Rahmen dieser Anmeldung verwendet, um deutlich zu machen, dass der Einsatz oder die Fraktion nicht ausschließlich aus Kohlenwasserstoffen mit der angegeben Kohlenstoffzahl besteht, sondern neben den Kohlenwasserstoffen der angegeben Kohlenstoffzahl auch Kohlenwasserstoffe mit anderen Kohlenstoffzahlen sowie andere Verunreinigungen vorhanden sein können. Bei der Trennung und Aufarbeitung des Produktstroms und/oder den Fraktionen bleiben stets Reste der Komponente(n) in dem Produktstrom beziehungsweise in der Fraktion. Auch andere Verunreinigungen bleiben bestehen, so dass ein bearbeiteter Produkt- oder Fraktionsstrom stets Rückstände enthält. Da der Aufwand für Abtrennung und Aufarbeitung mit der zu erzielenden Reinheit extrem stark ansteigt, hängt es von wirtschaftlichen Faktoren ab, welchen Anteil an Rückständen in einem Strom, den man entnimmt, um ihn beispielsweise rückzuführen, enthalten sein dürfen. Wie hoch dieser Anteil ist, muss nach wirtschaftlichen Gesichtspunkten abgewägt werden. Als groben Richtwert für den Anteil an unerwünschten Kohlenwasserstoffen und anderen Verunreinigungen wird in der Regel gelten, dass diese mit maximal 30 bis 40 Gewichtsprozent in dem Produktstrom und/oder in der Fraktion enthalten sein dürfen. Meist wird sogar ein Maximalwert von 15 Gewichtsprozent oder weniger erreicht. Für die rückgeführten Fraktionen gilt daher, dass diese die gewünschten Kohlenwasserstoffe mit mindestens 60 Gewichtsprozent, bevorzugt mindestens 80 Gewichtsprozent und weiter bevorzugt mindestens 90 Gewichtsprozent und besonders bevorzugt mindestens 95 Gewichtsprozent und ganz besonders bevorzugt mindestens 98 Gewichtsprozent enthalten.

Das Merkmal, dass der zweite Kohlenwasserstoffeinsatz größtenteils aus einer oder mehreren rückgeführten Fraktionen, welche aus dem Produktstrom abgetrennt werden, besteht, besagt, dass der oder die rückgeführten Fraktionen den größeren Teil des zweiten Kohlenwasserstoffeinsatzes ausmachen. Prinzipiell ist jedoch eine Zugabe eines Frischeinsatzes oder von anderen rückgeführten Fraktionen möglich, solange eine solche Zugabe nur von untergeordneter Bedeutung ist; d.h. das Verhalten des zweiten Kohlenwasserstoffes wird im Wesentlichen von den Eigenschaften rückgeführten Fraktionen gemäß Anspruch 1 bestimmt. Wie groß ein eventuell weiterer Anteil im zweiten Kohlenwasserstoffeinsatz sein darf, hängt folglich auch davon ab, inwieweit die Eigenschaften dieses eventuell weiter zugegebenen Anteils von den Eigenschaften der rückgeführten Fraktionen gemäß Anspruch 1 abweicht. Als Richtwert gilt aber, dass die rückgeführten Fraktionen gemäß Anspruch 1 mit mehr als der Hälfte überwiegen und vorzugsweise mehr als Dreiviertel des zweiten Kohlenwasserstoffeinsatzes ausmachen, besonders bevorzugt mehr als 90 Prozent und ganz besonders bevorzugt mehr als 95 Prozent des zweiten Kohlenwasserstoffeinsatzes. Insbesondere besteht der zweite Kohlenwasserstoffstrom ausschließlich aus einer oder mehreren rückgeführten Fraktionen, welche aus den Spaltproduktströmen gewonnen werden.

Die Vorgehensweisen, die notwendig sind, um den zweiten Kohlenwasserstoffeinsatz zu erhalten sind dem Fachmann bekannt. Es handelt sich um in Steamcrackern übliche Maßnahmen zum Abtrennen und Aufarbeiten von Produkt- und Fraktionsströmen.

Die Vorteile der Erfindung zeigen sich, wenn der zweite Kohlenwasserstoffeinsatz überwiegend Kohlenwasserstoffe mit einer Kohlenstoffzahl von 5 oder/und 4 enthält. Der zweite Kohlenwasserstoffeinsatz kann also überwiegend Kohlenwasserstoffen mit einer Kohlenstoffzahl von 5 oder aus Kohlenwasserstoffen mit einer Kohlenstoffzahl von 4 oder aus Gemischen von Kohlenwasserstoffen mit Kohlenstoffzahlen von 5 und von 4 enthalten. In vielen Anwendungsfällen sind die Gemische von Kohlenwasserstoffen mit Kohlenstoffzahlen von 5 und 4 als zweiter Kohlenwasserstoff von besonderem Vorteil.

Mit besonderem Vorteil enthält der zweite Kohlenwasserstoffeinsatz überwiegend gesättigte Kohlenwasserstoffe. Durch die Verwendung von überwiegend gesättigten Kohlenwasserstoffen als zweiten Kohlenwasserstoffeinsatz verbessert sich die thermische Dampfspaltung und es entsteht ein hoher Anteil an Wertprodukten. Diese Steigerung an Wertprodukten zeigt sich bei dem zweiten Kohlenwasserstoffeinsatz aufgrund seiner in Anspruch 1 festgelegten Kohlenstoffzahl besonders ausgeprägt. Damit überwiegend gesättigte Kohlenwasserstoffe als Einsatz verwendet werden können, muss vor der Rückführung eine Sättigung stattfinden. Dabei können nur die Fraktionen, die in den zweiten Kohlenwasserstoffeinsatz rückgeführt werden, abgesättigt werden oder es kann bereits an beliebiger Stelle vor der Abtrennung dieser Fraktionen eine Sättigung erfolgen. Die Methoden zur Abtrennung und zur Absättigung sind dem Fachmann bekannt und werden in Steamcracker üblicherweise eingesetzt.

In vorteilhafter Ausgestaltung der Erfindung ist der zweite Kohlenwasserstoff weitgehend frei von Diolefinen. Diolefine wirken sich im Spaltofen nachteilig aus. Dazu werden aus den Fraktionen, die in den zweiten Spaltofen rückgeführt werden, durch vorgeschaltete Umwandlungsverfahren oder Abtrennungsschritte die Diolefine überwiegend entfernt. Die Entfernung kann dabei entweder vor oder nach der Abtrennung der Fraktionen, die in den zweiten Spaltofen rückgeführt werden, erfolgen.

Insbesondere ist es von Vorteil, wenn die im zweiten Kohlenwasserstoffeinsatz enthaltenen Kohlenwasserstoffe mit einer Kohlenstoffzahl von 5 überwiegend gesättigte Kohlenwasserstoffe sind. Mit einem solchen Einsatz lässt sich der zweite Spaltofen vorteilhaft betreiben. Insbesondere ist solcher Einsatz besonders gut für eine Spaltung bei milden Bedingungen geeignet.

Für den ersten Kohlenwasserstoffeinsatz werden erfindungsgemäß die üblichen Einsätze verwendet (siehe Seite 1), für welchen die Spaltbedingungen des ersten Spaltofens sehr gut geeignet sind. Insbesondere ist der erste Spaltofen dazu geeignet langkettige Kohlenwasserstoffe umzusetzen. Es werden in den ersten Spaltofen daher auch Kohlenwasserstoffe mit einer Kohlenstoffzahl von 6 und mehr rückgeführt. Somit enthält der erste Kohlenwasserstoffeinsatz zumindest eine aus dem Produktstrom abgetrennte und rückgeführte Fraktion, welche überwiegend Kohlenwasserstoffe mit einer Kohlenstoffzahl von zumindest 6 aufweist.

Gemäß der Erfindung wird der zweite Kohlenwasserstoff im zweiten Spaltofen mit Spaltbedingungen umgesetzt, die zu einem Verhältnis von Propylen zu Ethylen bis 1,6 kg/kg führen. Bevorzugt wird der zweite Kohlenwasserstoff im zweiten Spaltofen mit Spaltbedingungen umgesetzt, die zu einem Verhältnis von Propylen zu Ethylen bis 1,4 kg/kg, besonders bevorzugt bis 1,2 kg/kg am Spaltofenaustritt führen. Wird der Einsatz bei milden Spaltbedingungen umgesetzt, zeigen sich die vorgenannten Vorteile der Erfindung besonders ausgeprägt. Von Vorteil sind auch Spaltbedingungen, die zu einem Verhältnis von Propylen zu Ethylen am Spaltofenaustritt führen von bis 1,5 kg/kg oder bis 1,15 kg/kg oder welche sogar in dem engen Bereich von 0,9 bis 1,1 kg/kg liegen.

Ferner wird der erste Kohlenwasserstoffeinsatz erfindungsgemäß mit Spaltbedingungen umgesetzt, die zu einem Verhältnis von Propylen zu Ethylen von 0,25 bis 0,85 kg/kg, bevorzugt von 0,3 bis 0,75 kg/kg, ebenfalls bevorzugt von 0,35 bis 0,7 kg/kg besonders bevorzugt von 0,4 bis 0,65 kg/kg am Spaltofenaustritt führen, wobei der Wert für das Verhältnis von Propylen zu Ethylen für den zweiten Kohlenwasserstoffeinsatz über dem Wert für das Verhältnis von Propylen zu Ethylen für den ersten Kohlenwasserstoffeinsatz liegt.

Durch das Betreiben von mindestens zwei Spaltöfen bei den eben genannten verschiedenen Spaltbedingungen stellen sich ganz besondere Vorteile ein, da die Spaltbedingungen in beiden Spaltöfen an den jeweiligen Einsatz angepasst werden kann. So zeichnet sich der zweite Kohlenwasserstoffeinsatz dadurch aus, dass mit diesem die angegebene sehr hohen Werte für das Verhältnis von Propylen zu Ethylen erreicht werden. Der erste Kohlenwasserstoffeinsatz wird hingegen bei üblichen Spaltbedingungen umgesetzt. Durch die Anpassung der Spaltbedingungen an ersten und zweiten Kohlenwasserstoffeinsatz wird erreicht, dass die Pyrolysebenzin-Fraktion mengenmäßig beherrschbar bleibt. Auch bildet der zweite Kohlenwasserstoffstoffeinsatz bei milden Bedingungen geringere Mengen an Pyrolyseöl als der erste Kohlenwasserstoffeinsatz. Für die thermische Dampfspaltung bei den ersten Spaltbedingungen im ersten Spaltofen hingegen wird ein üblicher Einsatz verwendet, welcher bei üblichen Spaltbedingungen beherrschbare Mengen an Pyrolysebenzin bildet.

Dabei liegen die Werte für das Verhältnis von Propylen zu Ethylen für ersten und zweiten Kohlenwasserstoff mit Vorteil um mindestens 0,1 kg/kg, vorzugsweise um mindestens 0,15 kg/kg und besonders bevorzugt um mindestens 0,2 kg/kg auseinander.

Wie eingangs erläutert, ergibt sich das Verhältnis von Propylen zu Ethylen beim thermischen Dampfspalten aus einer Reihe unterschiedlicher Einflussfaktoren, bei denen die Spaltofenaustrittstemperatur, d.h. die Temperatur eines Produktstroms beim Verlassen der verwendeten Reaktorschlange (engl. Coil Output Temperature), eine wichtige Rolle spielt. Die Spaltofenaustrittstemperatur für die Umsetzung im der zweiten Spaltofen liegt vorteilhafterweise zwischen 680 °C und 820 °C, bevorzugt zwischen 700 °C und 800 °C und weiter bevorzugt zwischen 710 °C und 780°C und besonders bevorzugt zwischen 720 °C und 760 °C. Die Spaltofenaustrittstemperatur für die Umsetzung im ersten Spaltofen liegt vorteilhafterweise zwischen 800 °C und 1000 °C, bevorzugt zwischen 820 °C und 950 °C und besonders bevorzugt zwischen 840 °C und 900 °C. Dabei ist die Spaltofenaustrittstemperatur am ersten Spaltofen stets höher als am zweiten Spaltofen.

Dabei liegt die Spaltofenaustrittstemperatur für die Umsetzung im ersten Spaltofen bevorzugt um mindestens 10 °C, besonders bevorzugt um mindestens 15 °C und ganz besonders bevorzugt um mindestens 20 °C über der Spaltofenaustrittstemperatur für die Umsetzung im zweiten Spaltofen.

Im zweiten Spaltofen kann ferner eine geringere Dampfverdünnung als im ersten verwendet werden. Dies verringert die notwendige Verdünnungsdampfmenge und spart Energie ein. Eine geringere Dampfverdünnung im zweiten Spaltofen ist jedoch nicht notwendig, damit sich die wesentlichen Vorteil der Erfindung zeigen. Vorteilhafterweise werden im zweiten Spaltofen 0,15 bis 0,8 kg Wasserdampf pro kg Kohlenwasserstoff im Einsatz verwendet während im ersten Spaltofen 0,3 bis 1,5 kg Wasserdampf pro kg Kohlenwasserstoff im Einsatz verwendet werden.

Auch können mit Vorteil in dem Produktstrom enthaltene, insbesondere gesättigte Kohlenwasserstoffe mit einer Kohlenstoffzahl von 2 bis 3 mit Vorteil mittels thermischen Dampfspalten in einem Spaltofen für gasförmigen Einsatz umgesetzt werden. Dazu werden die gesättigten gasförmigen Kohlenwasserstoffe aus dem Produktstrom gewonnen und in den Spaltofen für gasförmigen Einsatz rückgeführt und dort umgesetzt.

Als Frischeinsatz für den ersten Kohlenwasserstoffeinsatz können sowohl Gase oder Gasfraktionen wie Ethan, Propan oder Butan und entsprechende Gemische und Kondensate als auch flüssige Kohlenwasserstoffe und Kohlenwasserstoffgemische verwendet werden. Die genannten Gasgemische und Kondensate umfassen insbesondere sogenannte Erdgaskondensate (engl. Natural Gas Liquids, NGL). Die flüssigen Kohlenwasserstoffe und Kohlenwasserstoffgemische können beispielsweise aus der sogenannten Benzinfraktion von Rohöl stammen. Bei derartigen Rohbenzinen bzw. Naphthas (NT) und Kerosin handelt es sich um Gemische aus vorzugsweise gesättigten Verbindungen mit Siedepunkten zwischen 35 und 210 °C. Die Erfindung ist jedoch auch vorteilhaft beim Einsatz von Mitteldestillaten, atmosphärischen Rückständen und/oder hiervon abgeleiteten Gemischen aus der Rohölverarbeitung. Bei Mitteldestillaten handelt es sich um sogenannte leichte und schwere Gasöle, die als Ausgangsmaterialien zur Herstellung von leichten Heiz- und Dieselölen sowie von schwerem Heizöl verwendet werden können. Die enthaltenen Verbindungen weisen Siedepunkte von 180 bis 360 °C auf. Vorzugsweise handelt es sich um überwiegend gesättigte Verbindungen, die beim thermischen Dampfspalten umgesetzt werden können. Weiterhin können auch durch bekannte destillative Trennverfahren gewonnenen Fraktionen und entsprechenden Rückständen, aber auch die Verwendung jeweils hiervon, beispielsweise durch Hydrieren (engl. Hydrotreating) oder Hydrocracken, abgeleiteter Fraktionen verwendet werden. Beispiele sind Leicht-, Schwer- und Vakuumgasöl (engl. Atmospheric Gas Oil, AGO, bzw. Vacuum Gas Oil, VGO) sowie durch die genannten Hydrierverfahren behandelte Gemische und/oder Rückstände (engl. Hydrotreated Vacuum Gas Oil, HVGO, Hydrocracker Residue, HCR, bzw. Unconverted Oil, UCO).

Als Frischeinsatz für den ersten Kohlenwasserstoffeinsatz sind flüssige Kohlenwasserstoffe ganz besonders von Vorteil. Insbesondere werden als Frischeinsatz Erdgaskondensate und/oder Rohölfraktionen und/oder hiervon abgeleiteten Gemische verwendet.

Vorteilhafterweise umfasst die Erfindung damit die Verwendung von Kohlenwasserstoffgemischen mit einem Siedebereich von bis zu 600 °C als erstem Kohlenwasserstoffeinsatz als Frischeinsatz für den ersten Kohlenwasserstoffeinsatz. Innerhalb dieses Gesamtbereichs können auch Kohlenwasserstoffgemische mit abweichenden Siedebereichen verwendet werden, beispielsweise mit Siedebereichen von bis zu 360 °C oder von bis zu 240 °C. Die Reaktionsbedingungen im Spaltofen werden hierbei auf die jeweils eingesetzten Kohlenwasserstoffgemische abgestimmt. So kann die Erfindung mit Vorteil jedoch auch mit beliebigen anderen Frischeinsätzen verwendet werden, die vergleichbare Eigenschaften aufweisen, wie beispielsweise biogenen oder/und synthetische Kohlenwasserstoffe.

### Kurze Beschreibung der Zeichnung

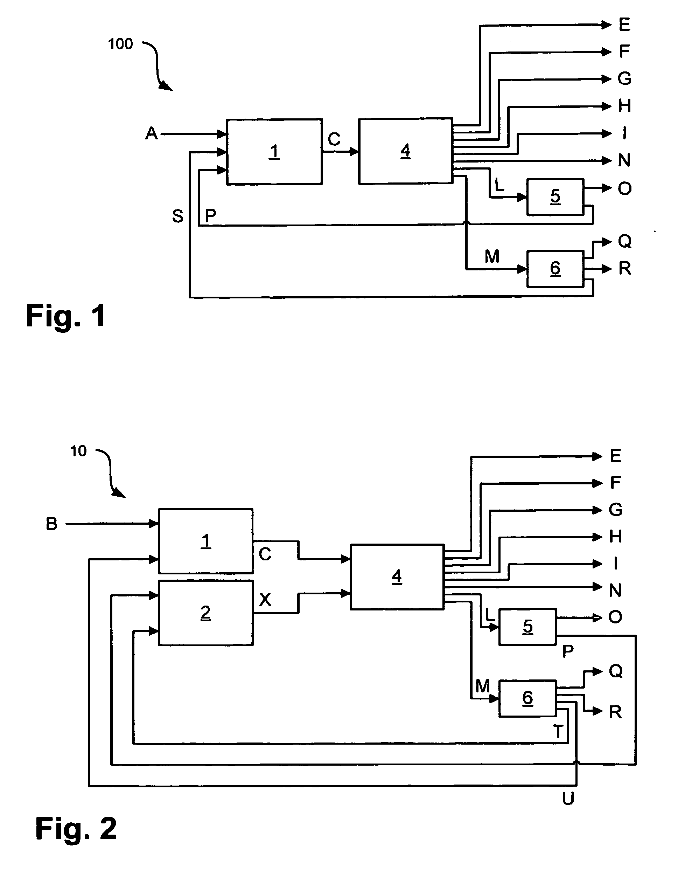

Das erfindungsgemäße Verfahren in besonders vorteilhafter Ausgestaltung soll anhand der Prozessschaubilder, welche die wesentlichen Prozessschritte schematisch zeigen, näher erklärt werden. Zum besseren Verständnis wird zuerst anhand von Figur 1 das bekannte Verfahren dargelegt.

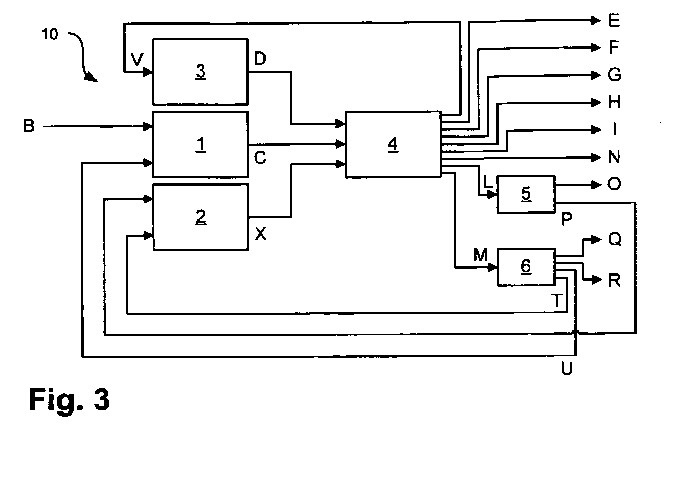

Figur 1 zeigt dazu in schematischer Darstellung ein bekanntes Vorgehen zur Olefinherstellung. Figur 2 zeigt in schematischer Darstellung die wesentlichen Schritte des erfindungsgemäßen Verfahrens in besonders vorteilhafter Ausgestaltung und Figur 3 zeigt, ebenfalls schematisch, die wesentlichen Schritte einer besonders vorteilhaften Ausgestaltung der Erfindung. In den Figuren tragen einander entsprechende Elemente identische Bezugzeichen.

Das schematische Prozessschaubild 100 der Figur 1 für das bekannte Verfahren beinhaltet einen Spaltofen 1, in welchen der Frischeinsatz A (beispielsweise Naphtha) sowie die rückgeführten Fraktionen S und P als Kohlenwasserstoffeinsatz geführt werden. Im Spaltofen 1 wird der Kohlenwasserstoffeinsatz in Konvektions- und Strahlungszone erwärmt und umgesetzt. In den Spaltofen wird Wasserdampf zugegeben, meist 0,5 bis 1 kg Prozessdampf pro kg Kohlenwasserstoff. Aus dem Spaltofen 1 tritt ein Produktstrom C aus, der direkt am Austritt aus dem Spaltofen auch als Spaltproduktstrom bezeichnet wird. Beim Austritt aus dem Spaltofen weist dieser Spaltproduktstrom eine Temperatur aus, die normalerweise zwischen 840 °C und 900°C liegt. Das Verhältnis von Propylen zu Ethylen liegt in der Regel bei 0,35 bis 0,6 kg/kg. Nach einem ersten Quenchen (nicht dargestellt) wird der Produktstrom wird in einer Aufarbeitungseinheit (englisch: processing unit) 4 verarbeitet. Aus der Aufarbeitungseinheit werden als wesentliche Produktfraktionen E bis N folgende Fraktionen gewonnen: Wasserstoff E, Ablauge F, Methan G, Ethylen H, Propylen I, gasförmige Kohlenwasserstoffe L mit einer Kohlenstoffzahl von 4, Pyrolysebenzin M und Pyrolyseöl N. Die gasförmigen Kohlenwasserstoffe L mit einer Kohlenwasserstoffzahl von 4 werden in einer C4-Aufarbeitungseinheit 5, welche für die Verarbeitung von Kohlenwasserstoffen mit einer Kohlenstoffzahl von 4 benutzt wird, weiter behandelt. Eine solche C4-Aufarbeitungseinheit 5 behandelt die Fraktion mit einer Kohlenstoffzahl von 4 derartig weiter, dass Butadien O abgeführt werden können. Die übrigen Kohlenwasserstoff mit einer Kohlenstoffzahl von 4 stellen eine Fraktion P dar, die in den Spaltofen 1 rückgeführt wird. Das Pyrolysebenzin M, welches Kohlenwasserstoffe mit einer Kohlenstoffzahl von 5 und mehr umfasst, wird in einer Pyrolysebenzin-Aufarbeitungseinheit 6 weiterverarbeitet und es werden Aromate Q und Kohlenwasserstoffe R mit einer Kohlenstoffzahl von beispielsweise mehr als 9 abgeführt. Die übrigen Kohlenwasserstoffe mit einer Kohlenstoffzahl von 5 und mehr werden als Fraktion S in den Spaltofen 1 rückgeführt. Die Aufarbeitungseinheit 4 sowie die C4-Aufarbeitungseinheit 5 und die Pyrolysebenzin-Aufarbeitungseinheit 6 umfassen übliche Einheiten zur Weiterverarbeitung des Produktstroms beziehungsweise der Produktfraktionen, welche zur Ausführung verschiedener Prozessschritte dienen, wie beispielsweise Verdichtung, Kondensation und Abkühlung, Trocknung, Destillation und Fraktionierung, Extraktion und Hydrierung. Die Prozessschritte sind in Olefinanlagen üblich und dem Fachmann bekannt.

Das schematische Prozessschaubild 10 der Figur 2 zeigt nun das erfindungsgemäße Verfahren in einer besonders vorteilhaften Ausgestaltung und seine wesentlichen Prozessschritte. Zusätzlich zu dem Spaltofen 1, in welchen der Frischeinsatz B (beispielsweise Naphtha) und die rückgeführte Fraktion U als ersten Kohlenwasserstoffeinsatz geführt wird, ist hier ein zweiter Spaltofen 2 vorhanden. In den zweiten Spaltofen 2 werden als zweiter Kohlenwasserstoffeinsatz die Fraktionen P und T geführt. Aus dem ersten Spaltofen 1 tritt wiederum der Spaltproduktstrom C mit den oben genannten Eigenschaften aus. Aus dem zweiten Spaltofen 2 tritt der Spaltproduktstrom X aus. Der Spaltproduktstrom X weist eine Temperatur aus, die vorteilhafterweise zwischen 700 °C und 800°C liegt. Das Verhältnis von Propylen zu Ethylen liegt dabei vorteilhafterweise zwischen 0,85 bis 1,5 kg/kg. Die Produktströme C und X werden in der Aufarbeitungseinheit 4 weiterverarbeitet und an geeigneter Stelle zu einem gemeinsamen Produktstrom zusammengeführt. Die Prozesse zur Weiterbehandlung und Aufarbeitung in der Aufarbeitungseinheit 4 sind bekannt und wurden eben beschrieben. So führt die Aufarbeitungseinheit 4 auch, wie eben beschrieben, zu den Produktfraktionen E bis N. Auch die Produktfraktionen L und M werden, wie eben beschrieben, in den speziellen Aufarbeitungseinheiten 5 und 6 weiterbehandelt. Im Gegensatz zu dem in Figur 1 beschriebenen Verfahren wird nun jedoch die Fraktion P, welche Kohlenwasserstoffe mit einer Kohlenstoffzahl von 4 enthält, nicht in den Spaltofen 1 sondern in den zweiten Spaltofen 2 rückgeführt. In der Pyrolysebenzin-Aufarbeitungseinheit 6 werden neben den oben erwähnten Fraktionen Q und R die Fraktionen T und U gewonnen. Die Fraktion T, welche Kohlenwasserstoffe mit einer Kohlenstoffzahl von 5 enthält, wird in den zweiten Spaltofen 2 rückgeführt, während die Fraktion U, welche Kohlenwasserstoffe mit einer Kohlenstoffzahl von 6 und mehr, insbesondere zwischen 6 und 9, enthält, in den ersten Spaltofen 1 rückgeführt wird.

Eine besonders vorteilhafte Ausgestaltung der Erfindung beinhaltet Figur 3. Figur 3 weist das gleiche schematische Prozessschaubild auf, wie es auch Figur 2 zeigt. Ergänzt ist dieses um einen Spaltofen 3 für gasförmigen Einsatz, in welche eine Fraktion V als Einsatz geführt wird. Die Fraktion V enthält gesättigte gasförmige Kohlenwasserstoffe mit einer Kohlenstoffzahl von 2 oder 3, welche ebenfalls in der Aufarbeitungseinheit 4 gewonnen werden.

### Bezugszeichenliste

- 1: Spaltofen (normale Spaltbedingungen)
- 2: Spaltofen (milde Spaltbedingungen)
- 3: Spaltofen für gasförmigen Einsatz
- 4: Aufarbeitungseinheit
- 5: C4-Aufarbeitungseinheit
- 6: Pyrolysebenzin-Aufarbeitungseinheit
- 10: schematisches Prozessschaubild für ein bekanntes Verfahren
- 100: schematisches Prozessschaubild für das erfindungsgemäße Verfahren in einer besonders vorteilhaften Ausgestaltung
- A, B: Frischeinsatz
- C, D, X: Produktströme
- E-V: Produktfraktionen

## Patentansprüche

1. Verfahren zur Umsetzung von Kohlenwasserstoffeinsätzen durch thermisches Dampfspalten zu einem olefinhaltigen Produktstrom, welcher zumindest Ethylen und Propylen enthält, wobei ein erster Kohlenwasserstoffeinsatz in mindestens einem ersten Spaltofen (1) und ein zweiter Kohlenwasserstoffeinsatz in mindesten einem zweiten Spaltofen (2) wenigstens teilweise umgesetzt wird, **dadurch gekennzeichnet, dass** der zweite Kohlenwasserstoffeinsatz überwiegend Kohlenwasserstoffe mit einer Kohlenstoffzahl von 5 oder/und 4 enthält und größtenteils aus einer oder mehreren rückgeführten Fraktionen (P, T), welche aus dem Produktstrom gewonnen werden, besteht, wobei der zweite Kohlenwasserstoff im zweiten Spaltofen (2) mit Spaltbedingungen umgesetzt wird, die zu einem Verhältnis von Propylen zu Ethylen von 0,85 bis 1,6 kg/kg führen, und dass dem ersten Kohlenwasserstoffeinsatz zumindest eine aus dem Produktstrom abgetrennte und rückgeführte Fraktion (U), welche überwiegend Kohlenwasserstoffe mit einer Kohlenstoffzahl von zumindest 6 aufweist, zugeführt wird, wobei der erste Kohlenwasserstoffeinsatz im ersten Spaltofen (1) mit Spaltbedingungen umgesetzt wird, die zu einem Verhältnis von Propylen zu Ethylen von 0,25 bis 0,85 kg/kg am Spaltofenaustritt führen und der Wert für das Verhältnis von Propylen zu Ethylen für den zweiten Kohlenwasserstoffeinsatz über dem Wert für das Verhältnis von Propylen zu Ethylen für den ersten Kohlenwasserstoffeinsatz liegt.

2. Verfahren nach Anspruch 1, **dadurch gekennzeichnet, dass** der zweite Kohlenwasserstoff ausschließlich aus einer oder mehreren rückgeführten Fraktionen (P, T) besteht.

3. Verfahren nach Anspruch 1 oder 2, **dadurch gekennzeichnet, dass** die im zweiten Kohlenwasserstoffeinsatz enthaltenen Kohlenwasserstoffe mit einer Kohlenstoffzahl von 5 überwiegend gesättigte Kohlenwasserstoffe sind.

4. Verfahren nach einem der Ansprüche 1 bis 3, **dadurch gekennzeichnet, dass** der zweite Kohlenwasserstoff weitgehend frei von Diolefinen ist.

5. Verfahren nach einem der Ansprüche 1 bis 4, **dadurch gekennzeichnet, dass** der zweite Kohlenwasserstoffeinsatz überwiegend gesättigte Kohlenwasserstoffe enthält.

6. Verfahren nach einem der Ansprüche 1 bis 5, **dadurch gekennzeichnet, dass** der zweite Kohlenwasserstoff im zweiten Spaltofen (2) mit Spaltbedingungen umgesetzt wird, die zu einem Verhältnis von Propylen zu Ethylen bis 1,2 kg/kg am Spaltofenaustritt führen.

7. Verfahren nach einem der Ansprüche 1 bis 6, **dadurch gekennzeichnet, dass** der der erste Kohlenwasserstoffeinsatz im ersten Spaltofen (1) mit Spaltbedingungen umgesetzt wird, die zu einem Verhältnis von Propylen zu Ethylen von 0,3 bis 0,75 kg/kg, bevorzugt von 0,4 bis 0,65 kg/kg am Spaltofenaustritt führen.

8. Verfahren nach einem der Ansprüche 1 bis 7, bei dem die Werte für das Verhältnis von Propylen zu Ethylen für ersten und zweiten Kohlenwasserstoff um mindestens 0,1 kg/kg, vorzugsweise um mindestens 0,15 kg/kg, besonders bevorzugt um mindestens 0,2 kg/kg auseinander liegen.

9. Verfahren nach einem der Ansprüche 1 bis 8, bei welchem die Spaltofenaustrittstemperatur für die Umsetzung im zweiten Spaltofen (2) zwischen 680 °C und 820 °C, bevorzugt zwischen 700 °C und 800 °C und weiter bevorzugt zwischen 710 °C und 780°C und besonders bevorzugt zwischen 720 °C und 760 °C liegt und die Spaltofenaustrittstemperaturfür die Umsetzung im ersten Spaltofen (1) zwischen 800 °C und 1000 °C, bevorzugt zwischen 820 °C und 950 °C und besonders bevorzugt zwischen 840 °C und 900 °C liegt, wobei die Spaltofenaustrittstemperatur des ersten Spaltofens (1) über der des zweiten Spaltofens (2) liegt.

10. Verfahren nach Anspruch 9, bei welchem die Spaltofenaustrittstemperatur für die Umsetzung im ersten Spaltofen (1) um mindestens 10 °C, bevorzugt um mindestens 15°C, besonders bevorzugt um mindestens 20 °C über der Spaltofenaustrittstemperatur für die Umsetzung im zweiten Spaltofen (2) liegt.

11. Verfahren nach einem der Ansprüche 1 bis 10, bei welchem im ersten Spaltofen (1) 0,3 bis 1,5 kg Wasserdampf pro kg Kohlenwasserstoffeinsatz und im zweiten Spaltofen (2) 0,15 bis 0,8 kg Wasserdampf pro kg Kohlenwasserstoffeinsatz verwendet wird.

12. Verfahren nach einem der Ansprüche 1 bis 11, bei welchem aus dem Produktstrom zumindest eine Fraktion (V), welche überwiegend Kohlenwasserstoffe mit einer Kohlenstoffzahl von 2 oder 3 aufweist, gewonnen wird und in einem Spaltofen (3) für gasförmigen Einsatz zumindest teilweise umgesetzt wird.

13. Verfahren nach einem der Ansprüche 1 bis 12, **dadurch gekennzeichnet, dass** als Frischeinsatz (B) für den ersten Kohlenwasserstoffeinsatz Erdgaskondensate und/oder Rohölfraktionen, insbesondere Naphtha, und/oder synthetische und/oder biogene Kohlenwasserstoffe und/oder hiervon abgeleiteten Gemische verwendet werden.

## Claims

1. Process for converting hydrocarbon inputs by thermal steamcracking to an olefin-containing product stream comprising at least ethylene and propylene, with at least partial conversion of a first hydrocarbon input in at least one first cracking furnace (1) and of a second hydrocarbon input in at least one second cracking furnace (2), **characterized in that** the second hydrocarbon input comprises predominantly hydrocarbons having a carbon number of 5 or/and 4 and consists for the most part of one or more recycled fractions (P, T) which are obtained from the product stream, the second hydrocarbon being converted in the second cracking furnace (2) with cracking conditions that lead to a ratio of propylene to ethylene of 0.85 to 1.6 kg/kg, and **in that** the first hydrocarbon input is supplied with at least one fraction (U) which has been separated from the product stream and recycled, comprising predominantly hydrocarbons having a carbon number of at least 6, the first hydrocarbon input being converted in the first cracking furnace (1) with cracking conditions that lead to a ratio of propylene to ethylene of 0.25 to 0.85 kg/kg at the cracking furnace outlet and the value for the ratio of propylene to ethylene for the second hydrocarbon input being above the value for the ratio of propylene to ethylene for the first hydrocarbon input.

2. Process according to Claim 1, **characterized in that** the second hydrocarbon consists exclusively of one or more recycled fractions (P, T).

3. Process according to Claim 1 or 2, **characterized in that** the hydrocarbons having a carbon number of 5 present in the second hydrocarbon input are predominantly saturated hydrocarbons.

4. Process according to any of Claims 1 to 3, **characterized in that** the second hydrocarbon is substantially free of diolefins.

5. Process according to any of Claims 1 to 4, **characterized in that** the second hydrocarbon input comprises predominantly saturated hydrocarbons.

6. Process according to any of Claims 1 to 5, **characterized in that** the second hydrocarbon is converted in the second cracking furnace (2) with cracking conditions that lead to a ratio of propylene to ethylene of up to 1.2 kg/kg at the cracking furnace outlet.

7. Process according to any of Claims 1 to 6, **characterized in that** the first hydrocarbon input is converted in the first cracking furnace (1) with cracking conditions that lead to a ratio of propylene to ethylene of 0.3 to 0.75 kg/kg, preferably of 0.4 to 0.65 kg/kg, at the cracking furnace outlet.

8. Process according to any of Claims 1 to 7, in which the values for the ratio of propylene to ethylene for the first and second hydrocarbons differ by at least 0.1 kg/kg, preferably by at least 0.15 kg/kg, more preferably by at least 0.2 kg/kg.

9. Process according to any of Claims 1 to 8, in which the cracking furnace exit temperature for the conversion in the second cracking furnace (2) is between 680°C and 820°C, preferably between 700°C and 800°C and further preferably between 710°C and 780°C and more preferably between 720°C and 760°C, and the cracking furnace exit temperature for the conversion in the first cracking furnace (1) is between 800°C and 1000°C, preferably between 820°C and 950°C and more preferably between 840°C and 900°C, the cracking furnace exit temperature of the first cracking furnace (1) being above that of the second cracking furnace (2) .

10. Process according to Claim 9, in which the cracking furnace exit temperature for the conversion in the first cracking furnace (1) is at least 10°C above, preferably at least 15°C above, more preferably at least 20°C above, the cracking furnace exit temperature for the conversion in the second cracking furnace (2).

11. Process according to any of Claims 1 to 10, in which 0.3 to 1.5 kg of steam per kg of hydrocarbon input is used in the first cracking furnace (1), and 0.15 to 0.8 kg of steam per kg of hydrocarbon input in the second cracking furnace (2).

12. Process according to any of Claims 1 to 11, in which at least one fraction (V) comprising predominantly hydrocarbons having a carbon number of 2 or 3 is obtained from the product stream and at least partly converted in a cracking furnace (3) for gaseous input.

13. Process according to any of Claims 1 to 12, **characterized in that** the fresh input (B) used for the first hydrocarbon input comprises natural gas condensates and/or crude oil fractions, especially naphtha, and/or synthetic and/or biogenic hydrocarbons and/or mixtures derived therefrom.

## Revendications

1. Procédé pour la transformation de charges hydrocarbonées par dissociation thermique à la vapeur en un flux de produits contenant des oléfines, qui contient au moins de l'éthylène et du propylène, une première charge hydrocarbonée étant au moins partiellement transformée dans au moins un premier four de dissociation (1) et une deuxième charge hydrocarbonée étant transformée au moins partiellement dans au moins un deuxième four de dissociation (2), **caractérisé en ce que** la deuxième charge hydrocarbonée contient principalement des hydrocarbures présentant un nombre de carbone de 5 et/ou 4 et est constituée, pour la plus grande partie, d'une ou de plusieurs fractions recyclées (P, T), qui sont obtenues à partir du flux de produits, le deuxième hydrocarbure étant transformé dans le deuxième four de dissociation (2) à des conditions de dissociation qui conduisent à un rapport de propylène à éthylène de 0,85 à 1,6 kg/kg, et **en ce que** la première charge hydrocarbonée est alimentée en au moins une fraction (U), séparée du flux de produits et recyclée, qui présente principalement des hydrocarbures présentant un nombre de carbone d'au moins 6, la première charge hydrocarbonée étant transformée dans le premier four de dissociation (1) à des conditions de dissociation qui conduisent à un rapport de propylène à éthylène de 0,25 à 0,85 kg/kg à la sortie du four de dissociation et la valeur pour le rapport de propylène à éthylène pour la deuxième charge hydrocarbonée étant supérieure à la valeur pour le rapport de propylène à éthylène pour la première charge hydrocarbonée.

2. Procédé selon la revendication 1, **caractérisé en ce que** le deuxième hydrocarbure est exclusivement constitué d'une ou de plusieurs fractions recyclées (P, T).

3. Procédé selon la revendication 1 ou 2, **caractérisé en ce que** les hydrocarbures contenus dans la deuxième charge hydrocarbonée présentant un nombre de carbone de 5 sont essentiellement des hydrocarbures saturés.

4. Procédé selon l'une quelconque des revendications 1 à 3, **caractérisé en ce que** le deuxième hydrocarbure est sensiblement exempt de dioléfines.

5. Procédé selon l'une quelconque des revendications 1 à 4, **caractérisé en ce que** la deuxième charge hydrocarbonée contient principalement des hydrocarbures saturés.

6. Procédé selon l'une quelconque des revendications 1 à 5, **caractérisé en ce que** le deuxième hydrocarbure est transformé dans le deuxième four de dissociation (2) à des conditions de dissociation qui conduisent à un rapport de propylène à éthylène de jusqu'à 1,2 kg/kg à la sortie du four de dissociation.

7. Procédé selon l'une quelconque des revendications 1 à 6, **caractérisé en ce que** la première charge hydrocarbonée est transformée dans le premier four de dissociation (1) à des conditions de dissociation qui conduisent à un rapport de propylène à éthylène de 0,3 à 0,75 kg/kg, de préférence de 0,4 à 0,65 kg/kg à la sortie du four de dissociation.

8. Procédé selon l'une quelconque des revendications 1 à 7, dans lequel les valeurs pour le rapport de propylène à éthylène pour le premier et le deuxième hydrocarbure sont écartées d'au moins 0,1 kg/kg, de préférence d'au moins 0,15 kg/kg, de manière particulièrement préférée d'au moins 0,2 kg/kg.

9. Procédé selon l'une quelconque des revendications 1 à 8, dans lequel la température à la sortie du four de dissociation pour la transformation dans le deuxième four de dissociation (2) est située entre 680°C et 820°C, de préférence entre 700°C et 800°C et plus préférablement entre 710°C et 780°C et de manière particulièrement préférée entre 720°C et 760°C et la température à la sortie du four de dissociation pour la transformation dans le premier four de dissociation (1) est située entre 800°C et 1000°C, de préférence entre 820°C et 950°C et de manière particulièrement préférée entre 840°C et 900°C, la température à la sortie du four de dissociation du premier four de dissociation (1) étant supérieure à celle du deuxième four de dissociation (2).

10. Procédé selon la revendication 9, dans lequel la température à la sortie du four de dissociation, pour la transformation dans le premier four de dissociation (1), est supérieure d'au moins 10°C, de préférence d'au moins 15°C, de manière particulièrement préférée d'au moins 20°C à la température à la sortie du four de dissociation pour la transformation dans le deuxième four de dissociation (2).

11. Procédé selon l'une quelconque des revendications 1 à 10, dans lequel on utilise, dans le premier four de dissociation (1), 0,3 à 1,5 kg de vapeur d'eau par kg de charge hydrocarbonée et, dans le deuxième four de dissociation (2), 0,15 à 0,8 kg de vapeur d'eau par kg de charge hydrocarbonée.

12. Procédé selon l'une quelconque des revendications 1 à 11, dans lequel on produit, à partir du flux de produits, au moins une fraction (V), qui présente essentiellement des hydrocarbures présentant un nombre de carbone de 2 ou 3 et on la transforme au moins partiellement dans un four de dissociation (3) pour une charge gazeuse.

13. Procédé selon l'une quelconque des revendications 1 à 12, **caractérisé en ce qu'**on utilise, comme charge fraîche (B) pour la première charge hydrocarbonée, des condensats de gaz naturel et/ou des fractions de pétrole brut, en particulier du naphta, et/ou des hydrocarbures de synthèse et/ou biogènes et/ou des mélanges dérivés de ceux-ci.
